# EUROPEAN PATENT SPECIFICATION

(11) **EP 3 185 912 B1**
(45) Date of publication and mention of the grant of the patent: **30.12.2020**
(21) Application number: 15748208.4
(22) Date of filing: 28.07.2015
(51) Int. Cl.: A61K 51/04, A61K 103/00

(54) **MONO-, DI- OR POLYSACCHARIDE USED AS METAL INHIBITOR IN THE PREPARATION OF 68GA-CHELATE-FUNCTIONALIZED TARGETING AGENT**
MONO-, DI- ODER POLYSACCHARID ALS METALLINHIBITOR ZUR HERSTELLUNG EINES 68GA-CHELATFUNKTIONALISIERTEN ZIELGERICHTETEN WIRKSTOFFES
MONOSACCHARIDE, DISACCHARIDE OU POLYSACCHARIDE UTILISÉ COMME INHIBITEUR DE MÉTAL DANS LA PRÉPARATION DE D'AGENT DE CIBLAGE FONCTIONNALISÉ PAR DU 68GA-CHÉLATE

(30) Priority: 29.08.2014 BE 201400653
(43) Date of publication of application: 05.07.2017
(73) Proprietor: Anmi S.A., 4000 Liège (BE)
(72) Inventor: WOUTERS, Ludovic, 4650 Herve (BE); KAISIN, Geoffroy, 4100 Seraing (BE); LUXEN, André, 4560 Ocquier - Clavier (BE); LÉONARD, Marc, 4400 Flémalle (BE); VOCCIA, Samuel, 4000 Liège (BE)
(74) Representative: De Clercq & Partners
(86) International application number: PCT/EP2015/067213
(87) International publication number: WO 2016/030104

(56) References cited:
- WO-A1-2010/092114
- WO-A2-03/059397
- WO-A2-2008/119036
- WO-A2-2013/024013
- US-A1- 2006 182 687
- US-A1- 2013 310 537
- EPPARD ELISABETH ET AL: "Ethanol-Based Post-processing of Generator-Derived Ga-68 Toward Kit-Type Preparation of Ga-68-Radiopharmaceuticals", JOURNAL OF NUCLEAR MEDICINE, vol. 55, no. 6, June 2014 (2014-06), pages 1023-1028, XP002750174,
- JAE YEON CHOI ET AL: "Development ofGa-labeled mannosylated human serum albumin (MSA) as a lymph node imaging agent for positron emission tomography", NUCLEAR MEDICINE AND BIOLOGY, ELSEVIER, NY, US, vol. 38, no. 3, 29 September 2010 (2010-09-29), pages 371-379, XP028190375, ISSN: 0969-8051, DOI: 10.1016/J.NUCMEDBIO.2010.09.010 [retrieved on 2010-10-08]
- M. I. M. Prata ET AL: "Targeting of lanthanide(III) chelates of DOTA-type glycoconjugates to the hepatic asyaloglycoprotein receptor: cell internalization and animal imaging studies", CONTRAST MEDIA & MOLECULAR IMAGING, vol. 1, no. 6, 1 January 2006 (2006-01-01) , pages 246-258, XP055520870, GB ISSN: 1555-4309, DOI: 10.1002/cmmi.111
- HAUBNER ROLAND ET AL: "Development of68Ga-labelled DTPA galactosyl human serum albumin for liver function imaging", EUROPEAN JOURNAL OF NUCLEAR MEDICINE AND MOLECULAR IMAGING, SPRINGER VERLAG, HEIDELBERG, DE, vol. 40, no. 8, 12 April 2013 (2013-04-12) , pages 1245-1255, XP035341691, ISSN: 1619-7070, DOI: 10.1007/S00259-013-2397-8 [retrieved on 2013-04-12]
- NEUMANN ET AL: "Native albumin for targeted drug delivery", EXPERT OPINION ON DRUG DELIVERY, INFORMA HEALTHCARE, GB, vol. 7, no. 8, 1 August 2010 (2010-08-01), pages 915-925, XP008170197, ISSN: 1742-5247, DOI: 10.1517/17425247.2010.498474
- VELIKYAN ET AL.: "Prospective of 68Ga-Radiopharmaceutical Development", THERANOSTIC, vol. 4, no. 1, 10 December 2013 (2013-12-10), pages 47-80,
- EIVAZIHOLLAGH ALIREZA ET AL: "Controlled Synthesis of Cu and Cu2O NPs and Incorporation of Octahedral Cu2O NPs in Cellulose II Films.", NANOMATERIALS (BASEL, SWITZERLAND) 14 APR 2018, vol. 8, no. 4, 14 April 2018 (2018-04-14), ISSN: 2079-4991

## Description

### TECHNICAL FIELD

The present invention is related to metal inhibitor improving radiolabelling yields and reliability

### BACKGROUND

Recently, some very interesting clinical results based on gallium-68 radiolabeled molecules for imaging in vivo by PET were published and presented. These radiopharmaceuticals are generally made of by assembly of a chelating agent with a targeting agent, generally DOTA-functionalized targeting agents, allowing, respectively, the reaction with a metallic radioisotope or radioactive metal and biological/metabolic activity of the radiopharmaceutical. This diagnostic isotope, i.e. gallium-68, has gained much interest because its substitution with lutecium-177 allows to switch from a diagnostic molecule to a therapeutic analogue readily usable for targeted radiotherapy. This "theranostic" (standing for diagnostic and therapeutic) field is in growth and will lead to major improvements in healthcare especially in oncology (J.Nucl.Med. 2011, 52, 841).

Among the radioactive metals usable for diagnostic or radiotherapy there is:
copper-64, gallium-68, gallium-67, gallium-66, lutecium-177, yttrium-86, yttrium-90, indium-114, indium-111, scandium-47, scandium-44, scandium-43, zirconium-89, bismuth-213, bismuth-212, actinium-225, lead-212, rhenium-188, rhenium-186, rubidium-82 and the like.

The labelling reaction with these radioactive metals is generally performed by chelating the radioactive metal with a suitable chelating agent in a suitable reaction medium, usually in a buffered medium in order to ensure an optimum pH for the chelation reaction.

However, these radioactive metals or their parent isotopes when issued from generators, are generally produced by cyclotron irradiation of either solid or liquid targets. Due to this production path these radioactive metals are generally not pure and contains some metallic byproducts.

The chelation reaction is dependent on a suitable pH, but on possible competition of the metallic impurities mentioned above with radioactive metals during the chelation reaction as well. In addition, it is generally accepted that heat can promotes the chelation reaction for the most commonly used radioactive metals based radiopharmaceuticals.

In the state of the art, the presence of metal ions that compete with radioactive metals is generally reduced by pre-labelling purification or fractionation of the radioactive but these additional steps represent a loss of radioactivity resulting from, either wasted time or the process itself. These losses can reach up to 30% of the total radioactivity.

The possibility of partial chelation of the radioactive metal requires, in general, a final post-labelling purification which allows to obtain a radiopharmaceutical having a radiochemical purity meeting the pharmaceutical specifications (> 90% radiochemical purity). These steps also represent an additional loss of activity that can raise up to 10% resulting from wasted time or the process itself.

According to known processes, at the end of the radiolabelling, a sequestering agent having a particular affinity for the unreacted radioactive metal may be added to chelate the non-reacted portion of the isotope. This complex formed by the sequestering agent and the non-reacted radioactive metal is then discarded in order to reach a better radiochemical purity after radiolabelling.

In addition, the need for these pre- and post-labelling purification steps makes these radioactive metal labelled radiopharmaceutical synthesis dependent, to some extent, on automation and on the use of a synthesis module. In addition to technical expertise, this requires extra time loss unfavorable to the overall performance.

Any improvement in order to achieve rapid, direct and high efficiency chelation is thus highly desirable.

Management of competing metal impurities is another challenge. Indeed, any species that would inhibit metal impurities by avoiding or having limited capacity to interfere negatively on the radioactive metal chelation reaction can act as a trap for these impurities. In other words, this inhibitory effect brings the apparent concentration of competitor metal, i.e. the concentration of metallic impurities yet available for chelation to a level which allows high yields and reproducible radiolabeling. This co-chelating agent is by definition different than the chelating agent assembled with the targeting agent.

It has been shown in WO2013024013 that adding a co-chelating agent could allow inhibition of competing metal impurities. Indeed, any species that would inhibit metal impurities by avoiding or having limited capacity to interfere negatively on the gallium-68 chelation reaction can act as a trap for these impurities.

US patent application 2013/310537 A1 discloses the synthesis of ⁶⁸Ga-labelled DOTA- and NODAGA-conjugated peptides carried at room temperature in an acetate buffer.

PCT patent application WO03/059397 discloses the synthesis of ⁶⁸Ga-labelled NOTA- and DOTA-conjugated peptides carried at room temperature in an ammonium acetate buffer.

In this context, it is clear that a need exists for an improved process for the preparation of radioactive metal complex which overcomes one or more of the above mentioned problems. This involves identifying an appropriate medium to handle the metal contamination, which if available avoids the need to heat for promoting the chelating reaction and allows radioactive metal chelation yields upper 90%. This heating is sometimes detrimental for the overall stability of the chelate-functionalized targeting agent

### SUMMARY

The scope of the present invention is defined by the appending claims. The invention disclosed herein is related to the use of metal inhibitors for improving radiolabelling yields and reliability of radioactive metal-based radiotracer synthesis, wherein the radiolabelling is performed with:
- A chelate-functionalized targeting agent, able to chelate the radioactive metal in the radiolabeling conditions
- A metal inhibitor, which is a co-chelating agent, capable of inactivating metals other than the radioactive metal without interfering with the chelation between the radioactive metal and the said chelate-functionalized targeting agent, under the conditions of the labelling reaction. In other words, said metal inhibitor is selected for its ability to chelate contaminating metals interfering and competing with the chelation of the radioactive metal while being mostly unable to chelate the radioactive metal in the said conditions of the labelling reaction as opposed to the chelate-functionalized targeting agent;
- A radioactive metal; and
- Optionally a buffer

The invention hence provides the following aspects:
Aspect 1. Use of a metal inhibitor for improving radiolabelling yields and reliability of radioactive metal-based radiotracer synthesis, wherein the radiolabelling is performed with:
   - a chelate-functionalized targeting agent, able to chelate the radioactive metal in the radiolabeling conditions, wherein the chelate functional group of the targeting agent is selected from the group comprising: DOTA, DOTAGA, TRITA, DO3A-Nprop, DTPA, tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, - NOPO, TETA, HBED, DFO, EDTA, 6SS, B6SS, PLED, TAME, YM103, NTP(PRHP)₃, H₂dedpa, H₂dp-bb-NCS, H₂dp-N-NCS; (4,6-MeO₂sal)₂-BAPEN and citrate;
   - a metal inhibitor selected from the group comprising: monosaccharides, disaccharides, and beta-cyclodextrin, which is a co-chelating agent, capable of inactivating metals other than the radioactive metal without interfering with the chelation between the radioactive metal and the said chelate-functionalized targeting agent, under the conditions of the labelling reaction;
   - a radioactive metal; and optionally
   - an acetate buffer allowing to maintain the pH in the range 3-8,
   wherein said targeting agent is a peptide consisting essentially of 2 to 20 amino acids, and
   wherein said metal inhibitor and chelate-functionalized targeting agent are not chemically linked.
Aspect 2. The use according to aspect 1, wherein said metal inhibitor is present in micromolar amounts, preferably in nanomolar amounts.
Aspect 3. The use according to aspect 1 or 2, wherein the radioactive metal is selected from the group comprising: copper-64, gallium-68, gallium-67, gallium-66, lutecium-177, yttrium-86, yttrium-90, indium-114, indium-111, scandium-47, scandium-44, scandium-43, zirconium-89, bismuth-213, bismuth-212, actinium-225, lead-212, rhenium-188, rhenium-186, and rubidium-82.
Aspect 4. The use according to any one of aspects 1 to 3, wherein the radioactive metal is a metal linked to a radioactive species.
Aspect 5. The use according to any one of aspects 1 to 3, wherein the radioactive metal is a fluorine-18 based metal fluoride.
Aspect 6. The use according to anyone of aspects 1 to 5, wherein said metal inhibitor is selected from the group comprising: Glucose, Fructose, and D-Mannose.
   Also disclosed is the use according to anyone of aspects 1 to 6, wherein said metal inhibitor and said functionalised agent are chemically linked, such as through a linker that is unstable in the radiolabelling conditions.
Aspect 7. The use according to anyone of aspects 1 to 6, wherein said radiolabelling is carried out at a temperature near or equal to room temperature.
Aspect 8. A method for radiolabelling a chelate-functionalized targeting agent with a metal radionuclide, comprising the steps of:
   a) providing a chelate-functionalized targeting agent, able to chelate the radioactive metal in the radiolabeling conditions, wherein the chelate functional group of the targeting agent is selected from the group comprising: DOTA, DOTAGA, TRITA, DO3A-Nprop, DTPA, tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, NOPO, TETA, HBED, DFO, EDTA, 6SS, B6SS, PLED, TAME, YM103, NTP(PRHP)₃, H₂dedpa, H₂dp-bb-NCS, H₂dp-N-NCS, (4,6-MeO₂sal)₂-BAPEN and citrate;
   b) adding a metal inhibitor to said targeting agent of a), said metal inhibitor being a co-chelating agent selected from the group comprising: monosaccharides, disaccharides, and beta-cyclodextrin and sulfated sugars, capable of inactivating metals other than the radioactive metal without interfering with the chelation between the radioactive metal and the said chelate-functionalized targeting agent, under the conditions of the labelling reaction; and
   c) adding a radioactive metal to the mixture of a) and b),
   in an acetate buffer allowing to maintain the pH in the range 3-8,
   wherein said targeting agent is a peptide consisting essentially of 2 to 20 amino acids, and
   wherein said metal inhibitor and chelate-functionalized targeting agent are not chemically linked.
Aspect 9. The method according to aspect 8, wherein said metal inhibitor is present in micromolar amounts, preferably in nanomolar amounts.
Aspect 10. The method according to any one of aspects 8 to 9, wherein the radiolabeling reaction is carried out at a temperature of below 50°C, preferably of ambient or room temperature (e.g. of between 20 and 30°C).
Aspect 11. The method according to any one of aspects 8 to 10, wherein the radiolabelling is performed at a pH comprised between 3 and 8, preferably between 3,5 and 7,5, more preferably between 3,5 and 7.
Aspect 12. The method according to any one of aspects 8 to 11, wherein the radioactive metal is selected from the group comprising: copper-64, gallium-68, gallium-67, gallium-66, lutecium-177, yttrium-86, yttrium-90, indium-114, indium-111, scandium-47, scandium-44, scandium-43, zirconium-89, bismuth-213, bismuth-212, actinium-225, lead-212, rhenium-188, rhenium-186, and rubidium-82; or wherein the radioactive metal is not gallium-68.
Aspect 13. The method according to any one of aspects 8 to 12, wherein the radioactive metal is a metal linked to a radioactive species.
Aspect 14. The method according to any one of aspects 8 to 13, wherein the radioactive metal is a fluorine-18 based metal fluoride.
Aspect 15. The method according to anyone of aspects 8 to 14, wherein said metal inhibitor is selected from the group comprising: Glucose, Fructose, and D-Mannose.
   Also disclosed are methods according to any one of aspects 8 to 15, wherein said metal inhibitor and said functionalised agent are chemically linked, such as through a linker that is unstable in the radiolabelling conditions.
   Also disclosed is a radiolabelled chelate-functionalized targeting agent obtained by the method according to anyone of aspects 8 to 15.
Aspect 16. A radiolabelling kit comprising:
   - a chelate-functionalized targeting agent, able to chelate the radioactive metal in the radiolabeling conditions, wherein the chelate functional group of the targeting agent is selected from the group comprising: DOTA, DOTAGA, TRITA, DO3A-Nprop, DTPA, tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, , NOPO, TETA, HBED, DFO, EDTA, 6SS, B6SS, PLED, TAME, YM103, NTP(PRHP)₃, H₂dp-bb-NCS, H₂dp-N-NCS, (4,6-MeO₂sal)₂-BAPEN, and citrate;
   - a metal inhibitor, which is a co-chelating agent selected from the group comprising: monosaccharides, disaccharides, and beta-cyclodextrin, capable of inactivating metals other than the radioactive metal without interfering with the chelation between the radioactive metal and the said chelate-functionalized targeting agent, under the conditions of the labelling reaction;
   - a radioactive metal; and
   - an acetate buffer. allowing to maintain the pH in the range 3-8,
   wherein said targeting agent is a peptide consisting essentially of 2 to 20 amino acids, and
   wherein said metal inhibitor and chelate-functionalized targeting agent are not chemically linked.
Aspect 17. The kit according to aspect 16, wherein said metal inhibitor is present in micromolar amounts, preferably in nanomolar amounts.
Aspect 18. The kit according to aspect 16 or 17, wherein the radioactive metal is selected from the group comprising: copper-64, gallium-68, gallium-67, gallium-66, lutecium-177, yttrium-86, yttrium-90, indium-114, indium-111, scandium-47, scandium-44, scandium-43, zirconium-89, bismuth-213, bismuth-212, actinium-225, lead-212, rhenium-188, rhenium-186, and rubidium-82.
Aspect 19. The kit according to any one of aspects 16 to 18, wherein the radioactive metal is a metal linked to a radioactive species.
Aspect 20. The kit according to any one of aspects 16 to 19, wherein the radioactive metal is a fluorine-18 based metal fluoride.
Aspect 21. The kit according to anyone of aspects 16 to 20, wherein said metal inhibitor is selected from the group comprising: Glucose, Fructose, and D-Mannose.
   Also disclosed are kits according to any one of aspects 16 to 21, wherein said metal inhibitor and said functionalised agent are chemically linked, such as through a linker that is unstable in the radiolabelling conditions.
Aspect 22. The kit according to any one of aspects 16 to 21, wherein said chelate-functionalized targeting agent and metal inhibitor are lyophilised.

### DETAILED DESCRIPTION

As used herein, the singular forms "a", "an", and "the" include both singular and plural referents unless the context clearly dictates otherwise.
The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1% or less of and from the specified value, insofar such variations are appropriate to perform in the present disclosure. It is to be understood that the value to which the modifier "about" refers is itself also specifically, and preferably, disclosed.

Whereas the term "one or more", such as one or more members of a group of members, is clear per se, by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise specified, all terms used herein, including technical and scientific terms, have the meaning as commonly understood by one of ordinary skill in the art to which this disclosure belongs. By means of further guidance, term definitions may be included to better appreciate the teaching of the present disclosure.

In the following passages, different aspects or embodiments of the disclosure are defined in more detail. Every aspect or embodiment so defined may be combined with each of the other aspects or embodiments unless stated otherwise. In particular, any feature indicated as being preferred or advantageous in one embodiment may be combined with any other embodiment or embodiments indicated as being preferred or advantageous.

The present disclosure overcomes one or more of the problems identified and observed in the state of the art and allows the direct radiolabeling of a chelate-functionalized targeting agents with radioactive metal.

The present disclosure is related to the use of a metal inhibitor for improving radiolabelling yields and reliability of radioactive metal-based radiotracer synthesis, the radiolabelling being performed with:
- A chelate-functionalized targeting agent, able to chelate the radioactive metal in the radiolabeling conditions;
- A metal inhibitor, which is a co-chelating agent, capable of inactivating metals other than the radioactive metal without interfering with the chelation between the radioactive metal and the said chelate-functionalized targeting agent, under the conditions of the labelling reaction. In other words, said metal inhibitor is selected for its ability to chelate contaminating metals interfering and competing with the chelation of the radioactive metal while being mostly unable to chelate the radioactive metal in the said conditions of the labelling reaction as opposed to the chelate-functionalized targeting agent;
- A radioactive metal; and
- Optionally a buffer.

Furthermore, it was found that a metal inhibitor can be used in the radiolabeling method for neutralizing, at least partially, interfering species and allowing the radioactive metal to react with the chelate-functionalized targeting agent. These metal inhibitors may temporarily or permanently remove metals that compete with radioactive metal for the reaction with the chelate-functionalized targeting agent. Said metal inhibitor is thus unable to chelate the radioactive metal in the said conditions of the labelling reaction, but chelates other metals interfering with the chelation of radioactive metal by the chelate-functionalized targeting agent. The presence of metal inhibitors during the radiolabeling reaction provides an advantageous alternative to current approaches for managing the presence of metallic impurities such as increasing the amount of chelate-functionalized targeting agent or the pretreatment of the eluate of the generator, these additional purification steps consume time (and radioactivity).

The aspects as described herein advantageously allow to obtain an appropriate chelation yield, particularly above 90 %, and therefore a sufficient radiochemical purity without any preliminary or further final purification.

The presence of a chelate-functionalized targeting agent, optionally a buffer and a metal inhibitor in the labelling medium advantageously allows to directly transfer the radioactive metal to the targeting agent and to perform the radiolabeling reaction without the need for any prior or subsequent operation or purification.

In addition, all kit components as described herein can be lyophilized altogether or frozen which ensures a longer shelf life.

Thus, the main advantages as disclosed herein that differentiate from the state of the art are:
- The possibility of radiolabeling without the need for an automated synthesizer;
- The possibility of a radiolabelling without the need for heating;
- The presence of a metal inhibitor which advantageously allows to use less chelate-functionalized targeting agent and allowing the implementation of more affordable radiopharmaceutical synthesis; and
- The presence of a metal inhibitor which advantageously allows improving the radiolabelling yields.

The metal inhibitors for use in the present invention are selected from the group comprising: monosaccharides, disaccharides, and beta-cyclodextrin.

Metal inhibitors disclosed herein are selected for their ability to block the competing metals in the radiolabelling reaction without inhibiting the radioactive metal ions in their chelation reaction with the chelate-functionalized targeting agent. Indeed, these metal inhibitors should not interfere negatively on the main radiolabeling reaction or lead to the formation of secondary radiolabeled species. In other words, metal inhibitors should have a limited or no capacity to complex radioactive metal in the conditions used for the radiolabelling reaction. Limited means at least 100 times less than the chelating agent used for the radiolabelling of the chelate-functionalized targeting agent.

It is interesting to note that the function of metal inhibitors as referred herein is the opposite of the function of the sequestering agents used in the prior art. Indeed, according to known methods, at the end of the labelling reaction, a sequestering agent having a particular affinity for e.g. the radioactive gallium may be added to chelate the unreacted portion of the isotope, whereas, according to the present disclosure an agent capable of reducing the competition of metallic impurities other than the radioactive metal is added at the beginning of the reaction.

As disclosed herein, an "inhibitor of metal" refers to any molecule capable of interacting with, or competing metals, or the chelating moiety of the chelate-functionalized targeting agent or with radioactive metal directly, to inhibit wholly or partially the chelation the chelate-functionalized targeting agent said competing metals and/or promote the chelating of radioactive metal by said targeting agent.

Examples of metal inhibitors disclosed herein are sugars that can be monosaccharides or derivatives of monosaccharides such as tetracetose, pentacetose, hexacétose, tetrose, pentose, hexose, D-mannose, D-fructose, and derivatives; and / or disaccharides and their derivatives such as maltose and its derivatives; and / or polysaccharides and their derivatives such as dextrins, cyclodextrins, sulfated sugars, cellulose and derivatives thereof.

Preferably, the metal inhibitor is present in the kit as described herein in micromolar amounts, preferably in nanomolar quantities, preferably in an amount of less than 500 nanomolar, still more preferably in an amount less than 100 nanomoles.

Also disclosed is the situation wherein the metal inhibitory agent is chemically bound to the chelate-functionalized targeting agent. This chemical bond can or cannot be a labile bond under the conditions of radiolabeling with the chelate-functionalized targeting agent like manner as in the conditions of radiolabeling the metal inhibitor is formed and released in situ.

As used herein, a "chelate-functionalized targeting agent" refers to a targeting agent capable of being labelled with a radioisotope such as for example radioactive metal, by means of a chelation agent to which this targeting agent is bound.

Examples of chelation agents for functionalizing a targeting agent to be radiolabeled with radioactive metals are those which form stable complexes at least for a time sufficient for diagnostic investigations using radiolabelled targeting agents, and include aliphatic amines, linear or macrocyclic such as macrocyclic amines with tertiary amines. Specific examples include: DOTA, DOTAGA, TRITA, DO3A-Nprop, DTPA, tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, NOPO, TETA, HBED, DFO, EDTA, 6SS, B6SS, PLED, TAME, YM103, NTP(PRHP)₃, H₂dedpa, H₂dp-bb-NCS, H₂dp-N-NCS, (4,6-MeO₂sal)₂-BAPEN and citrate.

The targeting agent that is chelate-functionalized for use in the current invention is a peptide consisting essentially of 2 to 20 amino acids.
A targeting agent as disclosed herein can be a peptide, a polypeptide, a protein, a vitamin, a saccharide, for example a monosaccharide or a polysaccharide, an antibody, nucleic acid, an aptamer, an antisense oligonucleotide, or an organic molecule.

Chelate-functionalized targeting agent as described herein preferably have a capacity of biological targeting. Examples of suitable targeting agents include molecules that target VEGF receptors, analogs of bombesin or GRP receptor targeting molecules, molecules targeting somatostatin receptors, RGD peptides or molecules targeting αvβ3 and αvβ5, annexin V or molecules targeting the apoptotic process, molecules targeting estrogen receptors, biomolecules targeting the plaque. More generally, a list targeting molecules, organic or not, functionalized by a chelating can be found in the journal of Velikyan et al., Theranostic 2014, Vol. 4, Issue 1 "Prospective of 68Ga-Radiopharmaceutical Development."

The term "radioactive metal" as used herein for radioactive labelling of the functionalised targeting agent(s) encompasses all radioactive metal ions suitable for use in medical imaging or radionuclides therapy. The radioactive metals are typically radioisotopes or radionuclides such as: copper-64, gallium-68, gallium-67, gallium-66, lutecium-177, yttrium-86, yttrium-90, indium-114, indium-111, scandium-47, scandium-44, scandium-43, zirconium-89, bismuth-213, bismuth-212, actinium-225, lead-212, rhenium-188, rhenium-186, rubidium-82 and the like. Many of these radionuclides are issued from nuclear reactor sub-products, cyclotron or from their specific radionuclide generator. In one embodiment, the radioactive metal is not gallium-68.

The term "radioactive metal" as used herein for radioactive labelling of the functionalised targeting agent(s) also encompasses metal linked to a radioactive species such as for example Fluorine-18-based metallic fluorides.

After addition of the radioactive metal solution to the metal inhibitor and the chelate-functionalized targeting agent, optionally containing a buffer, the solution obtained is left to the radiolabelling reaction for a short period of time, in particular between about 2 minutes and about 60 minutes, preferably from about 2 minutes to about 30 minutes, for example about 15 minutes.

Also disclosed is a radiolabelled targeting agent with radioactive metal, obtained by a method as described herein.

### Examples

### Example 1: Ga-68 Generator E & Z / NODAGA peptide without metal inhibitor:

### Labelling a peptide with a 68Ga eluate of 5 mL of 0.1 M HCl

A solution of 1850 MBGa-68 of 6(Eckert & Ziegler) is eluted with 5 mL of 0.1M HCI (Ultrapure grade) directly into a flask containing 150 mg of sodium acetate (Ultrapure grade) lyophilized, 240 µl of HCI 3M (Ultrapure grade), 760 µl of Milli-Q and 50 µg lyophilized NODAGA-NOC. The flask was left for 10 min at room temperature. The product is obtained with a radiochemical purity of 64% according to TLC analysis of the reaction medium.

Similarly to what was done in Example 1 different combinations were tested and are summarized in the table below:

| **#** | **Radioactive metal** | **Chelating agent use in the chelate-functionalized targeting agent** | **Metal Inhibitor** | **Labelling conditions** | **Radiolabelling yield vs radiolabelling yield without inhibitor** |
|---|---|---|---|---|---|
| 1 | Cu-64 | DOTA 25µg | Glucose | 10 minutes, 65°C | 82% vs 51% |
| 2 | Ga-68 | NODAGA 25µg | Fructose | 10 min, R.T. | 97% vs 61% |
| 3 | Ga68 | NOTA 25µg | Beta-cyclodextrin | 10 minutes, RT | 83% vs 51% |
| 4 | Sc-47 | DOTA 85µg | Beta-cyclodextrin | 30 minutes, 60°C | 85% vs 64% |
| 5 | Zr-89 | DFO | Beta-cyclodextrin | 30 minutes, RT | 91% vs 77% |
| 6 | Lu-177 | DOTA 100µg | Fructose | 30 minutes, 65°C | 94% vs 77% |
| 7 | In-111 | DOTA 100µg | Fructose | 30 minutes, 65°C | 85% vs 39% |
| 9 | Lu-177 | NODAGA 85µg | Beta-cyclodextrin | 30 minutes, 40°C | 95% vs 55% |
| 9 | Lu-177 | DOTA 100µg | Beta-cyclodextrin | 30 minutes, 40°C | 87% vs 51% |
| 10 | Ga-68 | NODAGA 25µg | D-Mannose | 10 minutes, RT | 97% vs 76% |
| 11 | Ga-68 | DOTA 100µg | D-Mannose | 30 minutes, RT | 80% vs 15% |
| 12 | Ga-68 | NODAGA 25µg | Fucoidan | 10 minutes, RT | 93% vs 76% |

Said targeting agent and metal inhibitor were present in a buffer consisting of phosphate, nitrate, HEPES, acetate, TRIS, ascorbate, or citrate, or a mixture thereof.

## Claims

1. Use of a metal inhibitor for improving radiolabelling yields and reliability of radioactive metal-based radiotracer synthesis, wherein the radiolabelling is performed with:
- a chelate-functionalized targeting agent, able to chelate the radioactive metal in the radiolabelling conditions, wherein the chelate functional group of the targeting agent is selected from the group comprising: DOTA, DOTAGA, TRITA, DO3A-Nprop, DTPA, tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, - NOPO, TETA, HBED, DFO, EDTA, 6SS, B6SS, PLED, TAME, YM103, NTP(PRHP)₃, H₂dedpa, H₂dp-bb-NCS, H₂dp-N-NCS; (4,6-MeO₂sal)₂-BAPEN and citrate;
- a metal inhibitor selected from the group comprising: monosaccharides, disaccharides, and beta-cyclodextrin, which is a co-chelating agent, capable of inactivating metals other than the radioactive metal without interfering with the chelation between the radioactive metal and the said chelate-functionalized targeting agent, under the conditions of the labelling reaction;
- a radioactive metal; and
- an acetate buffer allowing to maintain the pH in the range 3-8,
wherein said targeting agent is a peptide consisting essentially of 2 to 20 amino acids, and
wherein said metal inhibitor and chelate-functionalized targeting agent are not chemically linked.

2. The use according to claim 1, wherein said metal inhibitor is present in micromolar amounts, preferably in nanomolar amounts.

3. The use according to claim 1 or 2, wherein the radioactive metal is selected from the group comprising: copper-64, gallium-68, gallium-67, gallium-66, lutecium-177, yttrium-86, yttrium-90, indium-114, indium-111, scandium-47, scandium-44, scandium-43, zirconium-89, bismuth-213, bismuth-212, actinium-225, lead-212, rhenium-188, rhenium-186, and rubidium-82.

4. The use according to any one of claims 1 to 3, wherein the radioactive metal is a metal linked to a radioactive species, preferably wherein the radioactive metal is a fluorine-18 based metal fluoride.

5. The use according to anyone of claims 1 to 4, wherein said metal inhibitor is selected from the group comprising: Glucose, Fructose, and D-Mannose.

6. The use according to anyone of claims 1 to 5, wherein said radiolabelling is carried out at a temperature near or equal to room temperature.

7. A method for radiolabelling a chelate-functionalized targeting agent with a metal radionuclide, comprising the steps of:
a) providing a chelate-functionalized targeting agent, able to chelate the radioactive metal in the radiolabeling conditions, wherein the chelate functional group of the targeting agent is selected from the group comprising: DOTA, DOTAGA, TRITA, DO3A-Nprop, DTPA, tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, NOPO, TETA, HBED, DFO, EDTA, 6SS, B6SS, PLED, TAME, YM103, NTP(PRHP)₃, H₂dedpa, H₂dp-bb-NCS, H₂dp-N-NCS, (4,6-MeO₂sal)₂-BAPEN and citrate;
b) adding a metal inhibitor to said targeting agent of a), said metal inhibitor being a co-chelating agent selected from the group comprising: monosaccharides, disaccharides, and beta-cyclodextrin, capable of inactivating metals other than the radioactive metal without interfering with the chelation between the radioactive metal and the said chelate-functionalized targeting agent, under the conditions of the labelling reaction; and
c) adding a radioactive metal to the mixture of a) and b.
in an acetate buffer allowing to maintain the pH in the range 3-8,
wherein said targeting agent is a peptideconsisting essentially of 2 to 20 amino acids, and
wherein said metal inhibitor and chelate-functionalized targeting agent are not chemically linked.

8. The method according to claim 7, wherein said metal inhibitor is present in micromolar amounts, preferably in nanomolar amounts.

9. The method according to any one of claims 7 to 8, wherein the radiolabeling reaction is carried out at a temperature of below 50°C, preferably of ambient or room temperature (e.g. of between 20 and 30°C).

10. The method according to any one of claims 7 to 9, wherein the radiolabelling is performed at a pH comprised between 3 and 8, preferably between 3,5 and 7,5, more preferably between 3,5 and 7.

11. The method according to any one of claims 7 to 10, wherein the radioactive metal is selected from the group comprising: copper-64, gallium-68, gallium-67, gallium-66, lutecium-177, yttrium-86, yttrium-90, indium-114, indium-111, scandium-47, scandium-44, scandium-43, zirconium-89, bismuth-213, bismuth-212, actinium-225, lead-212, rhenium-188, rhenium-186, and rubidium-82.

12. The method according to any one of claims 7 to 11, wherein the radioactive metal is a metal linked to a radioactive species, preferably wherein the radioactive metal is a fluorine-18 based metal fluoride.

13. The method according to anyone of claims 7 to 12, wherein said metal inhibitor is selected from the group comprising: Glucose, Fructose, and D-Mannose.

14. A radiolabelling kit comprising:
- a chelate-functionalized targeting agent, able to chelate the radioactive metal in the radiolabeling conditions, wherein the chelate functional group of the targeting agent is selected from the group comprising: DOTA, DOTAGA, TRITA, DO3A-Nprop, DTPA, tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, , NOPO, TETA, HBED, DFO, EDTA, 6SS, B6SS, PLED, TAME, YM103, NTP(PRHP)₃, 2-H₂dedpa, H₂dp-bb-NCS, H₂dp-N-NCS, (4,6-MeO₂sal)₂-BAPEN, and citrate;
- a metal inhibitor, which is a co-chelating agent selected from the group comprising: monosaccharides, disaccharides, and beta-cyclodextrin, capable of inactivating metals other than the radioactive metal without interfering with the chelation between the radioactive metal and the said chelate-functionalized targeting agent, under the conditions of the labelling reaction;
- a radioactive metal; and
- an acetate buffer. allowing to maintain the pH in the range 3-8,
wherein said targeting agent is a peptide consisting essentially of 2 to 20 amino acids, and
wherein said metal inhibitor and chelate-functionalized targeting agent are not chemically linked.

15. The kit according to claim 14, wherein said metal inhibitor is present in micromolar amounts, preferably in nanomolar amounts.

16. The kit according to claim 14 or 15, wherein the radioactive metal is selected from the group comprising: copper-64, gallium-68, gallium-67, gallium-66, lutecium-177, yttrium-86, yttrium-90, indium-114, indium-111, scandium-47, scandium-44, scandium-43, zirconium-89, bismuth-213, bismuth-212, actinium-225, lead-212, rhenium-188, rhenium-186, and rubidium-82.

17. The kit according to any one of claims 14 to 16, wherein the radioactive metal is a metal linked to a radioactive species, preferably wherein the radioactive metal is a fluorine-18 based metal fluoride.

18. The kit according to anyone of claims 14 to 17, wherein said metal inhibitor is selected from the group comprising: Glucose, Fructose, and D-Mannose.

19. The kit according to any one of claims 14 to 18, wherein said chelate-functionalized targeting agent and metal inhibitor are lyophilised.

## Patentansprüche

1. Verwendung eines Metallhemmers zur Verbesserung von Ausbeuten bei radioaktiver Markierung und Zuverlässigkeit der Synthese von Radiotracer auf Metallbasis, wobei die radioaktive Markierung durchgeführt wird mit:
- einem mit einer Chelatfunktion versehenen Targeting-Mittel, das bei den Bedingungen der radioaktiven Markierung ein Chelat mit dem radioaktiven Metall bilden kann, wobei die Chelatfunktionsgruppe des Targeting-Mittels ausgewählt ist aus der Gruppe umfassend: DOTA, DOTAGA, TRITA, DO3A-Nprop, DTPA, Tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, NOPO, TETA, HBED, DFO, EDTA, 6SS, B6SS, PLED, TAME, YM103, NTP(PRHP)₃, H₂dedpa, H₂dp-bb-NCS, H₂dp-N-NCS, (4,6-MeO₂sal}₂-BAPEN und Citrat;
- einem Metallhemmer ausgewählt aus der Gruppe umfassend: Monosaccharide, Disaccharide und Beta-Cyclodextrin, bei dem es sich um einen Co-Chelatbildner handelt, der andere Metalle als das radioaktive Metall unter den Bedingungen der Markierungsreaktion inaktivieren kann, ohne die Chelatbildung zwischen dem radioaktiven Metall und dem mit einer Chelatfunktion versehenen Targeting-Mittel zu stören;
- einem radioaktiven Metall; und
- einem Acetatpuffer, der Halten des pH-Werts im Bereich 3-8 gestattet,
wobei es sich bei dem Targeting-Mittel um ein Peptid handelt, das im Wesentlichen aus 2 bis 20 Aminosäuren besteht, und
wobei der Metallhemmer und das mit einer Chelatfunktion versehene Targeting-Mittel nicht chemisch verknüpft sind.

2. Verwendung nach Anspruch 1, wobei der Metallhemmer in mikromolaren Mengen, bevorzugt in nanomolaren Mengen vorliegt.

3. Verwendung nach Anspruch 1 oder 2, wobei das radioaktive Metall ausgewählt ist aus der Gruppe umfassend: Kupfer-64, Gallium-68, Gallium-67, Gallium-66, Lutetium-177, Yttrium-86, Yttrium-90, Indium-114, Indium-111, Scandium-47, Scandium-44, Scandium-43, Zirconium-89, Bismut-213, Bismut-212, Actinium-225, Blei-212, Rhenium-188, Rhenium-186 und Rubidium-82.

4. Verwendung nach einem der Ansprüche 1 bis 3, wobei es sich bei dem radioaktiven Metall um ein mit einer radioaktiven Spezies verknüpftes Metall handelt, vorzugsweise wobei es sich bei dem radioaktiven Metall um ein auf Fluor-18 basierendes Metallfluorid handelt.

5. Verwendung nach einem der Ansprüche 1 bis 4, wobei der Metallhemmer ausgewählt ist aus der Gruppe umfassend: Glucose, Fructose und D-Mannose.

6. Verwendung nach einem der Ansprüche 1 bis 5, wobei die radioaktive Markierung bei einer Temperatur nahe oder gleich Raumtemperatur durchgeführt wird.

7. Verfahren zur radioaktiven Markierung eines mit einer Chelatfunktion versehenen Targeting-Mittels mit einem Metall-Radionuklid, umfassend die Schritte:
a) Bereitstellen eines mit einer Chelatfunktion versehenen Targeting-Mittels, das bei den Bedingungen der radioaktiven Markierung ein Chelat mit dem radioaktiven Metall bilden kann, wobei die Chelatfunktionsgruppe des Targeting-Mittels ausgewählt ist aus der Gruppe umfassend: DOTA, DOTAGA, TRITA, DO3A-Nprop, DTPA, Tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, NOPO, TETA, HBED, DFO, EDTA, 6SS, B6SS, PLED, TAME, YM103, NTP(PRHP)₃, H₂dedpa, H₂dp-bb-NCS, H₂dp-N-NCS; (4,6-MeO₂sal}₂-BAPEN und Citrat;
b) Zugeben eines Metallhemmers zum Targeting-Mittel unter a), wobei es sich bei dem Metallhemmer um einen Co-Chelatbildner handelt, der ausgewählt ist aus der Gruppe umfassend: Monosaccharide, Disaccharide und Beta-Cyclodextrin, der andere Metalle als das radioaktive Metall unter den Bedingungen der Markierungsreaktion inaktivieren kann, ohne die Chelatbildung zwischen dem radioaktiven Metall und dem mit einer Chelatfunktion versehenen Targeting-Mittel zu stören; und
c) Zugeben eines radioaktiven Metalls zum Gemisch unter a) und b;
in einem Acetatpuffer, der Halten des pH-Werts im Bereich 3-8 gestattet,
wobei es sich bei dem Targeting-Mittel um ein Peptid handelt, das im Wesentlichen aus 2 bis 20 Aminosäuren besteht, und
wobei der Metallhemmer und das mit einer Chelatfunktion versehene Targeting-Mittel nicht chemisch verknüpft sind.

8. Verfahren nach Anspruch 7, wobei der Metallhemmer in mikromolaren Mengen, bevorzugt in nanomolaren Mengen vorliegt.

9. Verfahren nach einem der Ansprüche 7 bis 8, wobei die Reaktion zur radioaktiven Markierung bei einer Temperatur unter 50°C, vorzugsweise bei Umgebungs- oder Raumtemperatur (z. B. zwischen 20 und 30°C), durchgeführt wird.

10. Verfahren nach einem der Ansprüche 7 bis 9, wobei die radioaktive Markierung bei einem zwischen 3 und 8, bevorzugt zwischen 3,5 und 7,5, stärker bevorzugt zwischen 3,5 und 7 liegenden pH-Wert erfolgt.

11. Verfahren nach einem der Ansprüche 7 bis 10, wobei das radioaktive Metall ausgewählt ist aus der Gruppe umfassend: Kupfer-64, Gallium-68, Gallium-67, Gallium-66, Lutetium-177, Yttrium-86, Yttrium-90, Indium-114, Indium-111, Scandium-47, Scandium-44, Scandium-43, Zirconium-89, Bismut-213, Bismut-212, Actinium-225, Blei-212, Rhenium-188, Rhenium-186 und Rubidium-82.

12. Verfahren nach einem der Ansprüche 7 bis 11, wobei es sich bei dem radioaktiven Metall um ein mit einer radioaktiven Spezies verknüpftes Metall handelt, vorzugsweise wobei es sich bei dem radioaktiven Metall um ein auf Fluor-18 basierendes Metallfluorid handelt.

13. Verfahren nach einem der Ansprüche 7 bis 12, wobei der Metallhemmer ausgewählt ist aus der Gruppe umfassend: Glucose, Fructose und D-Mannose.

14. Kit zur radioaktiven Markierung, umfassend:
- ein mit einer Chelatfunktion versehenes Targeting-Mittel, das bei den Bedingungen der radioaktiven Markierung ein Chelat mit dem radioaktiven Metall bilden kann, wobei die Chelatfunktionsgruppe des Targeting-Mittels ausgewählt ist aus der Gruppe umfassend: DOTA, DOTAGA, TRITA, DO3A-Nprop, DTPA, Tetra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, NOPO, TETA, HBED, DFO, EDTA, 6SS, B6SS, PLED, TAME, YM103, NTP(PRHP)₃, H₂dedpa, H₂dp-bb-NCS, H₂dp-N-NCS; (4,6-MeO₂sal}₂-BAPEN und Citrat;
- einen Metallhemmer, bei dem es sich um einen Co-Chelatbildner ausgewählt aus der Gruppe umfassend: Monosaccharide, Disaccharide und Beta-Cyclodextrin handelt, der andere Metalle als das radioaktive Metall unter den Bedingungen der Markierungsreaktion inaktivieren kann, ohne die Chelatbildung zwischen dem radioaktiven Metall und dem mit einer Chelatfunktion versehenen Targeting-Mittel zu stören;
- ein radioaktives Metall; und
- einen Acetatpuffer, der Halten des pH-Werts im Bereich 3-8 gestattet,
wobei es sich bei dem Targeting-Mittel um ein Peptid handelt, das im Wesentlichen aus 2 bis 20 Aminosäuren besteht, und
wobei der Metallhemmer und das mit einer Chelatfunktion versehene Targeting-Mittel nicht chemisch verknüpft sind.

15. Kit nach Anspruch 14, wobei der Metallhemmer in mikromolaren Mengen, bevorzugt in nanomolaren Mengen vorliegt.

16. Kit nach Anspruch 14 oder 15, wobei das radioaktive Metall ausgewählt ist aus der Gruppe umfassend: Kupfer-64, Gallium-68, Gallium-67, Gallium-66, Lutetium-177, Yttrium-86, Yttrium-90, Indium-114, Indium-111, Scandium-47, Scandium-44, Scandium-43, Zirconium-89, Bismut-213, Bismut-212, Actinium-225, Blei-212, Rhenium-188, Rhenium-186 und Rubidium-82.

17. Kit nach einem der Ansprüche 14 bis 16, wobei es sich bei dem radioaktiven Metall um ein mit einer radioaktiven Spezies verknüpftes Metall handelt, vorzugsweise wobei es sich bei dem radioaktiven Metall um ein auf Fluor-18 basierendes Metallfluorid handelt.

18. Kit nach einem der Ansprüche 14 bis 17, wobei der Metallhemmer ausgewählt ist aus der Gruppe umfassend: Glucose, Fructose und D-Mannose.

19. Kit nach einem der Ansprüche 14 bis 18, wobei das mit einer Chelatfunktion versehene Targeting-Mittel und der Metallhemmer lyophilisiert sind.

## Revendications

1. Utilisation d'un inhibiteur de métal pour améliorer les rendements d'un radio-marquage et la fiabilité d'une synthèse de radio-traceur basée sur un métal radioactif, dans laquelle le radio-marquage est exécuté avec :
- un agent de ciblage fonctionnalisé par chélate, capable de chélater le métal radioactif dans les conditions du radio-marquage, dans lequel le groupe fonctionnel chélate de l'agent de ciblage est choisi dans le groupe comprenant les : DOTA, DOTAGA, TRITA, DO3A-Nprop, DTPA, tétra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, NOPO, TETA, HBED, DFO, EDTA, 6SS, B6SS, PLED, TAME, YM103, NTP(PRHP)₃, H₂dedpa, H₂dp-bb-NCS, H₂dp-N-NCS , (4,6-MeO₂sal)₂-BAPEN et citrate ;
- un inhibiteur de métal choisi dans le groupe comprenant des : monosaccharides, disaccharides et bêta-cyclodextrine, qui est un co-agent chélateur, capable d'inactiver des métaux autres que le métal radioactif sans interférer avec la chélation entre le métal radioactif et ledit agent de ciblage fonctionnalisé par chélate, dans les conditions de la réaction de marquage ;
- un métal radioactif ; et
- un tampon d'acétate permettant de maintenir le pH dans la plage des 3-8,
dans laquelle ledit agent de ciblage est un peptide constitué essentiellement par 2 à 20 acides aminés, et
dans laquelle lesdits inhibiteur de métal et agent de ciblage fonctionnalisé par chélate ne sont pas chimiquement liés.

2. Utilisation selon la revendication 1, dans laquelle lequel ledit inhibiteur de métal est présent en quantités micro-molaires, de préférence en quantités nano-molaires.

3. Utilisation selon les revendications 1 ou 2, dans laquelle le métal radioactif est choisi dans le groupe comprenant les : cuivre-64, gallium-68, gallium-67, gallium-66, lutécium-177, yttrium-86, yttrium-90, indium-114, indium-111, scandium-47, scandium-44, scandium-43, zirconium-89, bismuth-213, bismuth-212, actinium-225, plomb-212, rhénium-188, rhénium-186 et rubidium-82.

4. Utilisation selon l'une quelconque des revendications 1 à 3, dans laquelle le métal radioactif est un métal lié à une espèce radioactive, de préférence dans laquelle le métal radioactif est un fluorure de métal à base de fluor 18.

5. Utilisation selon l'une quelconque des revendications 1 à 4, dans laquelle ledit inhibiteur de métal est choisi dans le groupe comprenant les : glucose, fructose et D-mannose.

6. Utilisation selon l'une quelconque des revendications 1 à 5, dans laquelle ledit radio-marquage est effectué à une température proche de la température de la pièce ou égale à celle-ci.

7. Procédé destiné à radio-marquer un agent de ciblage fonctionnalisé par chélate avec un radionucléide de métal, comprenant les étapes de :
a) fourniture d'un agent de ciblage fonctionnalisé par chélate, capable de chélater le métal radioactif dans les conditions du radio-marquage, dans lequel le groupe fonctionnel chélate de l'agent de ciblage est choisi dans le groupe comprenant les : DOTA, DOTAGA, TRITA, DO3A-Nprop, DTPA, tétra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, NOPO, TETA, HBED, DFO, EDTA, 6SS, B6SS, PLED, TAME, YM103, NTP(PRHP)₃, H₂dedpa, H₂dp-bb-NCS, H₂dp-N-NCS, (4,6-MeO₂sal)₂-BAPEN et citrate ;
b) ajout d'un inhibiteur de métal au dit agent de ciblage de l'étape a), ledit inhibiteur de métal étant un agent co-chélateur choisi dans le groupe comprenant des : monosaccharides, disaccharides et bêta-cyclodextrine, capable d'inactiver des métaux autres que le métal radioactif sans interférer avec la chélation entre le métal radioactif et ledit agent de ciblage fonctionnalisé par chélate, dans les conditions de la réaction de marquage ; et
c) ajout d'un métal radioactif au mélange des étapes a) et b ;
dans un tampon d'acétate permettant de maintenir le pH dans la plage des 3-8,
dans lequel ledit agent de ciblage est un peptide constitué essentiellement par 2 à 20 acides aminés, et dans lequel lesdits inhibiteur de métal et agent de ciblage fonctionnalisé par chélate ne sont pas chimiquement liés.

8. Procédé selon la revendication 7, dans lequel ledit inhibiteur de métal est présent en quantités micro-molaires, de préférence en quantités nano-molaires.

9. Procédé selon l'une quelconque des revendications 7 à 8, dans lequel la réaction de radio-marquage est effectuée à une température en-dessous des 50°C, de préférence à la température ambiante ou de la pièce (à titre d'exemple, comprise entre 20 et 30°C).

10. Procédé selon l'une quelconque des revendications 7 à 9, dans lequel le radio-marquage est exécuté à un pH compris entre 3 et 8, de préférence entre 3,5 et 7,5, mieux préféré entre 3,5 et 7.

11. Procédé selon l'une quelconque des revendications 7 à 10, dans lequel le métal radioactif est choisi dans le groupe comprenant les : cuivre-64, gallium-68, gallium-67, gallium-66, lutécium-177, yttrium-86, yttrium-90, indium-114, indium-111, scandium-47, scandium-44, scandium-43, zirconium-89, bismuth-213, bismuth-212, actinium-225, plomb-212, rhénium-188, rhénium-186 et rubidium-82.

12. Procédé selon l'une quelconque des revendications 7 à 11, dans lequel le métal radioactif est un métal lié à une espèce radioactive, de préférence dans lequel le métal radioactif est un fluorure de métal à base de fluor 18.

13. Procédé selon l'une quelconque des revendications 7 à 12, dans lequel ledit inhibiteur de métal est choisi dans le groupe comprenant les : glucose, fructose et D-mannose.

14. Trousse de radio-marquage comprenant :
- un agent de ciblage fonctionnalisé par chélate, capable de chélater le métal radioactif dans les conditions du radio-marquage, dans laquelle le groupe fonctionnel chélate de l'agent de ciblage est choisi dans le groupe comprenant les : DOTA, DOTAGA, TRITA, DO3A-Nprop, DTPA, tétra-tBu-DTPA, p-SCN-Bz-DTPA, MX-DTPA, CHX-DTPA, NOTA, TACN, TACN-TM, H3NOKA, NODASA, NODAGA, NOTP, NOTPME, PrP9, TRAP, NOPO, TETA, HBED, DFO, EDTA, 6SS, B6SS, PLED, TAME, YM103, NTP(PRHP)₃, H₂dedpa, H₂dp-bb-NCS, H₂dp-N-NCS, (4,6-MeO₂sal)₂-BAPEN et citrate ;
- un inhibiteur de métal, qui est un co-agent chélateur choisi dans le groupe comprenant des : monosaccharides, disaccharides et bêta-cyclodextrine, capable d'inactiver des métaux autres que le métal radioactif sans interférer avec la chélation entre le métal radioactif et ledit agent de ciblage fonctionnalisé par chélate, dans les conditions de la réaction de marquage ;
- un métal radioactif ; et
- un tampon d'acétate permettant de maintenir le pH dans la plage des 3-8,
dans laquelle ledit agent de ciblage est un peptide constitué essentiellement par 2 à 20 acides aminés, et
dans laquelle lesdits inhibiteur de métal et agent de ciblage fonctionnalisé par chélate ne sont pas chimiquement liés.

15. Trousse selon la revendication 14, dans laquelle ledit inhibiteur de métal est présent en quantités micro-molaires, de préférence en quantités nano-molaires.

16. Trousse selon les revendications 14 ou 15, dans laquelle le métal radioactif est choisi dans le groupe comprenant les : cuivre-64, gallium-68, gallium-67, gallium-66, lutécium-177, yttrium-86, yttrium-90, indium-114, indium-111, scandium-47, scandium-44, scandium-43, zirconium-89, bismuth-213, bismuth-212, actinium-225, plomb-212, rhénium-188, rhénium-186 et rubidium-82.

17. Trousse selon l'une quelconque des revendications 14 à 16, dans laquelle le métal radioactif est un métal lié à une espèce radioactive, de préférence dans laquelle le métal radioactif est un fluorure de métal à base de fluor 18.

18. Trousse selon l'une quelconque des revendications 14 à 17, dans laquelle ledit inhibiteur de métal est choisi dans le groupe comprenant les : glucose, fructose et D-mannose.

19. Trousse selon l'une quelconque des revendications 14 à 18, dans laquelle lesdits agent de ciblage fonctionnalisé par chélate et inhibiteur de métal sont lyophilisés.
